# EUROPEAN PATENT APPLICATION

(11) **EP 2 589 358 A1**
(43) Date of publication of application: **08.05.2013**
(21) Application number: 11800717.8
(22) Date of filing: 23.06.2011
(51) Int. Cl.: A61F 13/49, A61F 13/15, A61F 13/494, A61F 13/56

(54) **DISPOSABLE DIAPERS**

(30) Priority: 30.06.2010 JP 2010150648
(71) Applicant: Unicharm Corporation, Ehime 799-0111 (JP)
(72) Inventor: OHASHI, Naoto, Kanonji-shi Kagawa 769-1602 (JP); KIKUCHI, Kyo, Kanonji-shi Kagawa 769-1602 (JP); YAMANAKA, Yasuhiro, Kanonji-shi Kagawa 769-1602 (JP)
(74) Representative: Peter, Julian
(86) International application number: PCT/JP2011/064417
(87) International publication number: WO 2012/002252

(57) **Abstract**

A disposable wearing article improved so that a front waist region of a chassis can be reliably folded outwardly by relatively simple handling and, in such state, the article can be reliably fitted to the wearer's body. An article chassis 11 is provided with an absorbent core 22 extending across a crotch region 14 into front and rear waist regions 12, 13 and the absorbent core 22 is formed with a fold guiding zone 24 extending across the front waist region 12 in a direction of a transverse axis Q. A fastening system includes a first fastening system composed of first fastening elements 17 provided on respective skin-contactable surfaces of a pair of fastening members extending outward in the direction of the transverse axis Q from lateral edges 13a, 13b of the rear waist region 13 and a second fastening system composed of the first fastening elements 17 and a third fastening element 45 formed on the skin-contactable surface of the front waist region 12.

## Description

### {Technical Field}

The present invention relates to disposable wearing articles and more particularly to open-type disposable articles such as open-type disposable diapers primarily for newborn babies, disposable toilet-training pants, disposable incontinent pants or disposable sanitary pants.

### {Background}

Conventionally, disposable wearing articles for newborn babies are widely known. For example, JP 3343199 B2 (PTL 1) discloses a disposable diaper including a liquid-permeable topsheet, a liquid-impermeable backsheet, an absorbent core interposed between the top- and backsheets and a pair of wearing bands extending circumferentially of wearer's waist.

### {Citation List}

### {Patent Literature}

. {PTL 1}: JP 3343199 B2

### {Summary}

### {Technical Problem}

The disposable diaper disclosed in PTL 1 includes a fold guiding zone formed at a predetermined location in a front end portion of a diaper chassis so as to extend in the transverse direction of the diaper with an arrangement such that a thickness of the absorbent core is smaller in a zone corresponding to the fold guiding zone than in the adjacent zone or the absorbent core is not present in this zone.

The front end portion of the diaper chassis may be folded outwardly along the fold guiding zone and the upper end of the wearing band to leave a navel of a newborn baby as the wearer not covered with the diaper and, in consequence, abdominal respiration of the wearer should not be impeded. In addition, the folded portion can be secured to the diaper chassis by the intermediary of fastening means provided on the outer surface of the front end portion and thereby the navel can be reliably exposed.

However, the front end portion of the diaper chassis is folded outwardly along the fold guiding zone and the upper edge of the wearing band after the diaper has been put on the wearer's body with use of the wearing band. With such a wearing mode, if it has been perceived that the wearer's navel is exposed just and no more in the vicinity of the fold guiding zone or even positioned below the fold guiding zone, it is required to unfasten the wearing band and to readjust the position of the diaper. Further, in order to prevent the portion once having been folded outwardly from being rolled up, any other means to fasten the folded portion will be required in addition to the wearing band and consequently the handling for fastening may take a lot of trouble. Furthermore, the wearing band having a relatively large width dimension may tighten the wearer's waist and the wearer would experience an uncomfortable feeling of compression.

An object of the present invention is to provide a disposable wearing article by improving the conventional invention so that a front waist region of a chassis can be reliably folded outwardly by relatively simple handling and, in such a state, the article can be reliably fitted to the wearer's body.

### {Solution to Problem}

The present invention includes first and second aspects and provides a disposable wearing article having a longitudinal axis and a transverse axis and including a chassis and a fastening system. The chassis includes a skin-contactable surface, a non-skin-contactable surface opposite to the skin-contactable surface, a front waist region, a rear waist region and a crotch region extending in a direction of the longitudinal axis between the front and rear waist regions. The fastening system is adapted to join respective lateral edges of the front and rear waist regions.

In the first aspect of the present invention, the disposable wearing article includes the following features:
the chassis is provided with an absorbent core extending across the crotch region into the front and rear waist regions;
the absorbent core is formed with a fold guiding zone extending in the direction of the transverse axis in the front waist region; and
the fastening system includes a first fastening system and a second fastening system, wherein the first fastening system includes first fastening elements provided on the respective skin-contactable surfaces of a pair of tape fastener tabs extending outward in the direction of the transverse axis from lateral edges of the rear waist region and a second fastening element formed on the non-skin-contactable surface of the front waist region, and the second fastening system includes the pair of the first fastening elements and a third fastening element formed on the skin-contactable surface of the front waist region.

In the second aspect of the present invention, the disposable wearing article includes the following features:
the chassis is provided with an absorbent core extending across the crotch region into the front and rear waist regions;
the absorbent core is formed with a fold guiding zone extending in the direction of the transverse axis in the front waist region;
the fastening system includes a first fastening system and a second fastening system, wherein the first fastening system includes first fastening elements provided on the respective skin-contactable surfaces of a pair of tape fastener tabs extending outward in the direction of the transverse axis from lateral edges of the rear waist region and a second fastening element formed on the non-skin-contactable surface of the front waist region, and
a second fastening system including the pair of the first fastening elements and a third fastening element formed on the skin-contactable surface of the front waist region; and
the second fastening element is located on the non-skin-contactable surface of the front waist region so as to be closer to the waist-opening than to the fold guiding zone and a sub-region in the front waist region extending outward from the fold guiding zone in the direction of the transverse axis may be folded outwardly to engage the first fastening elements with the third fastening element.

According to one embodiment of the present invention in the first aspect thereof, the absorbent core in the crotch region is formed with a crotch fold guiding zone extending along the transverse axis.

According to another embodiment of the present invention in the first aspect thereof, the crotch fold guiding zone is formed on the absorbent core in the crotch region at a location biased toward the rear waist region.

According to even another embodiment of the present invention in the first aspect thereof, the fold guiding zone and the crotch fold guiding zone are provided in the form of grooves.

According to still another embodiment of the present invention in the first aspect thereof, the absorbent core is not present in the fold guiding zone.

According to yet another embodiment of the present invention in the first aspect thereof, the third fastening element is formed by embossing a fibrous nonwoven fabric sheet defining the skin-contactable surface of the front waist region.

According to one embodiment of the present invention in the second aspect thereof, the absorbent core in the crotch region is formed with a crotch fold guiding zone extending along the transverse axis.

According to another embodiment of the present invention in the second aspect thereof, the crotch fold guiding zone is formed on the absorbent core in the crotch region at a location biased toward the rear waist region.

According to even another embodiment of the present invention in the second aspect thereof, the fold guiding zone and the crotch fold guiding zone are provided in the form of grooves.

According to still another embodiment of the present invention in the second aspect thereof, the absorbent core is not present in the fold guiding zone.

According to yet another embodiment of the present invention in the second aspect thereof, the third fastening element is formed by embossing a fibrous nonwoven fabric sheet defining the skin-contactable surface of the front waist region.

According to a further embodiment of the present invention in the second aspect thereof, the non-skin-contactable surface of the front waist region is provided in a zone corresponding to the fold guiding zone with a fold guiding line adapted to be visually recognized from the outside.

### {Advantageous Effects of Invention}

According to the present invention, the fastening system used to join the respective lateral edges of the front and rear waist regions is composed of first and second fastening systems and therefore these fastening systems may be used in an appropriately selective manner depending on the wearer's body size and thereby a size control of the diaper is significantly facilitated. When the second fastening system is utilized, the wearer's abdominal region is unlikely to be tightened over a relatively large area. This effect is beneficial particularly for newborn babies, because their abdominal respiration should not be impeded.

### {Brief Description of Drawings}

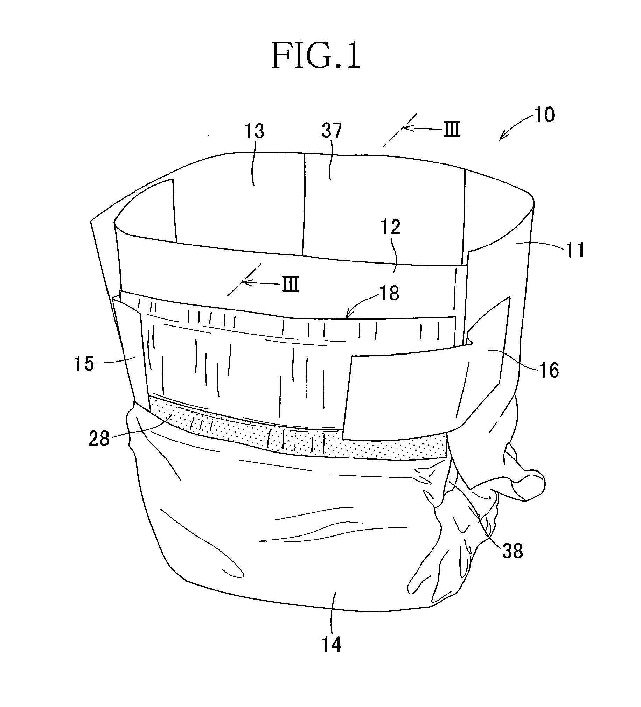

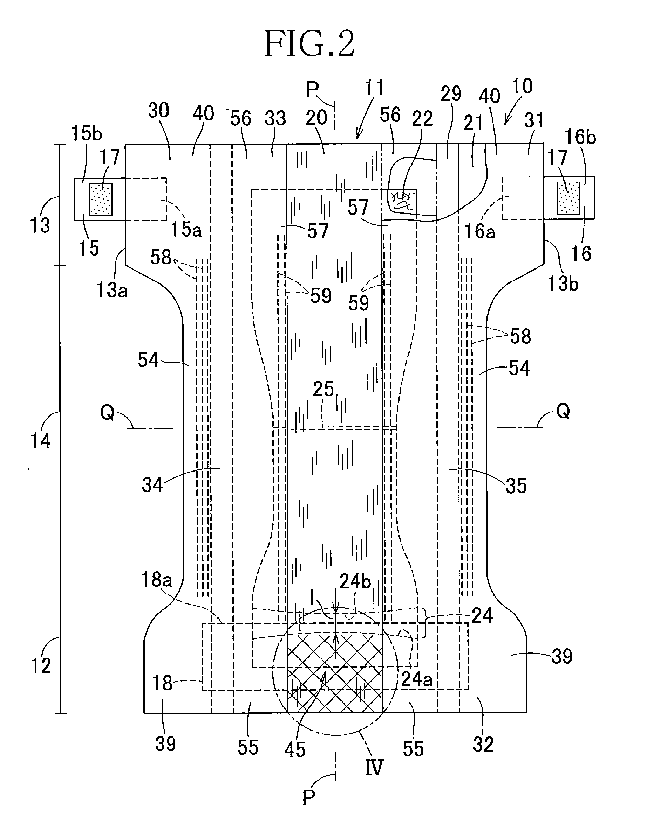

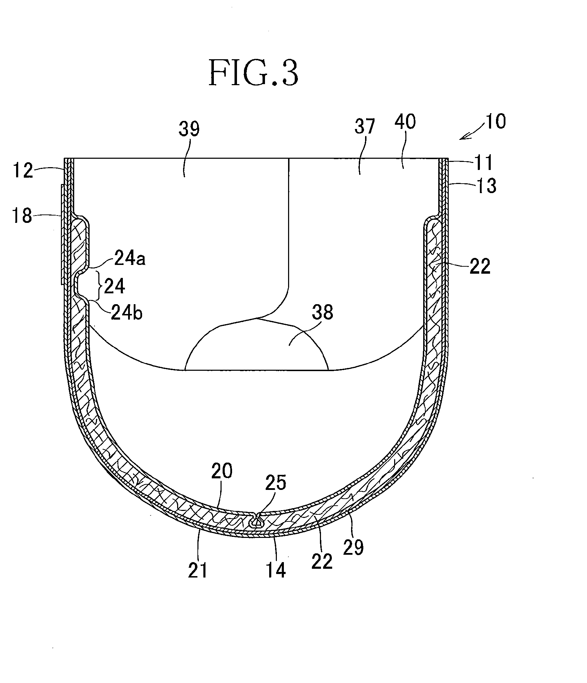

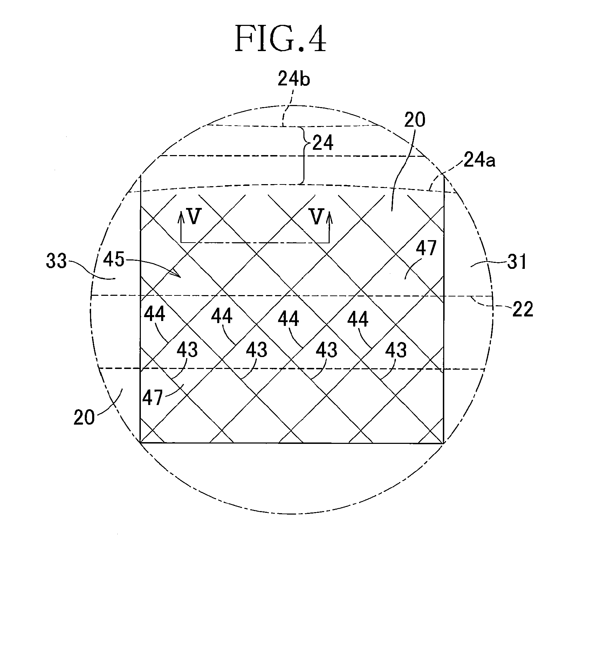

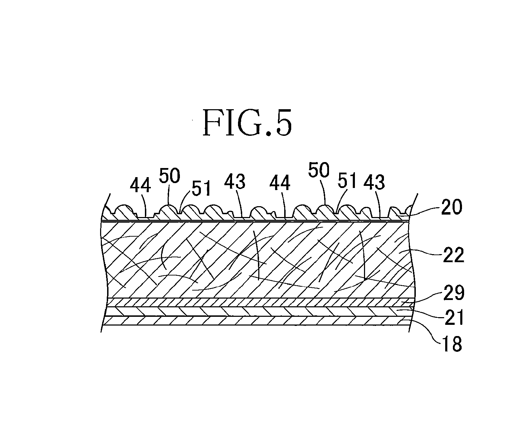

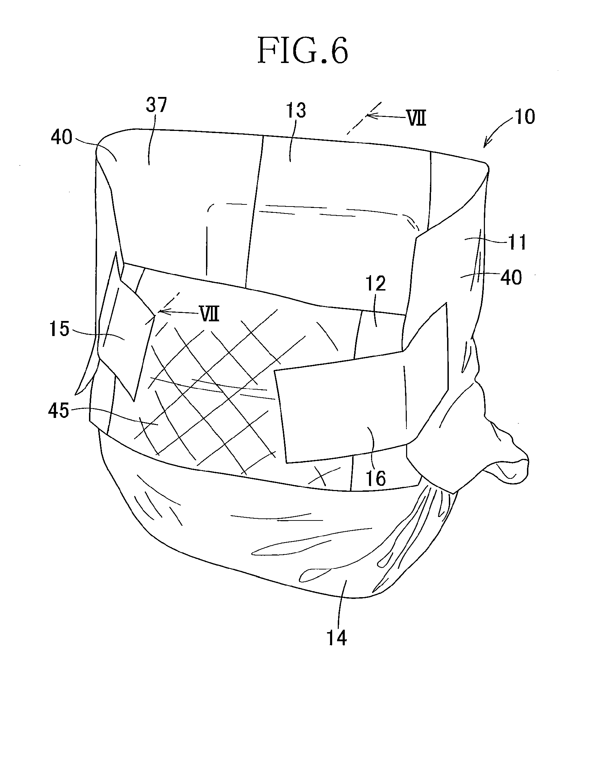

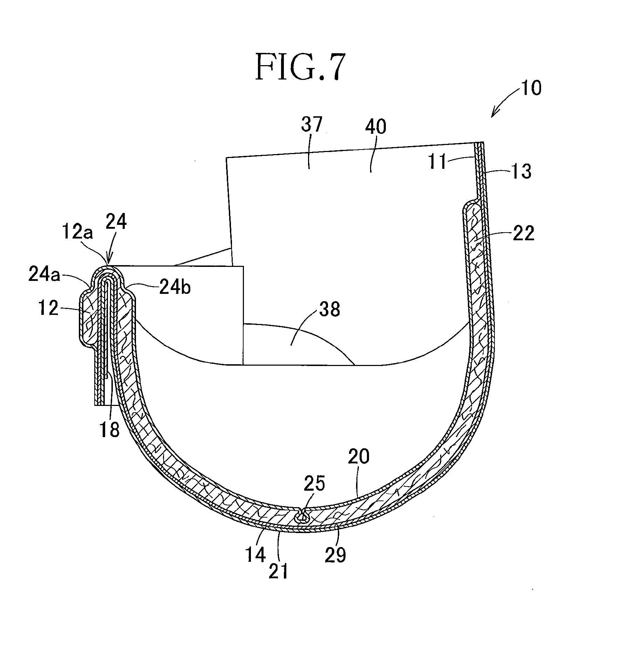

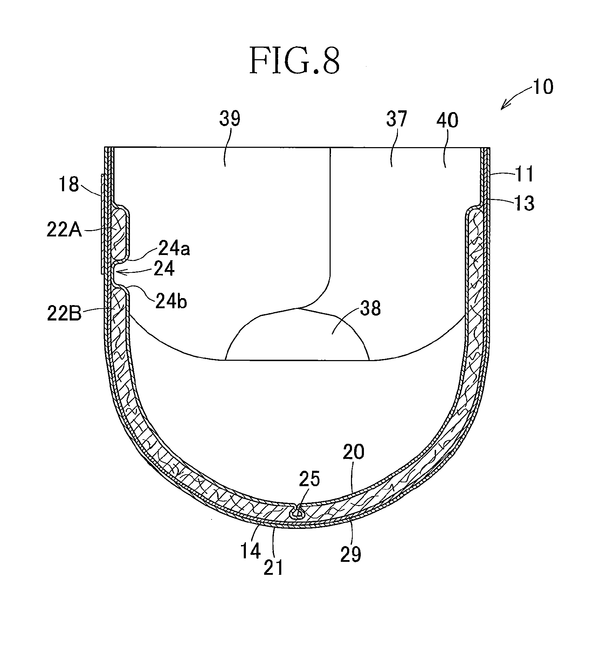

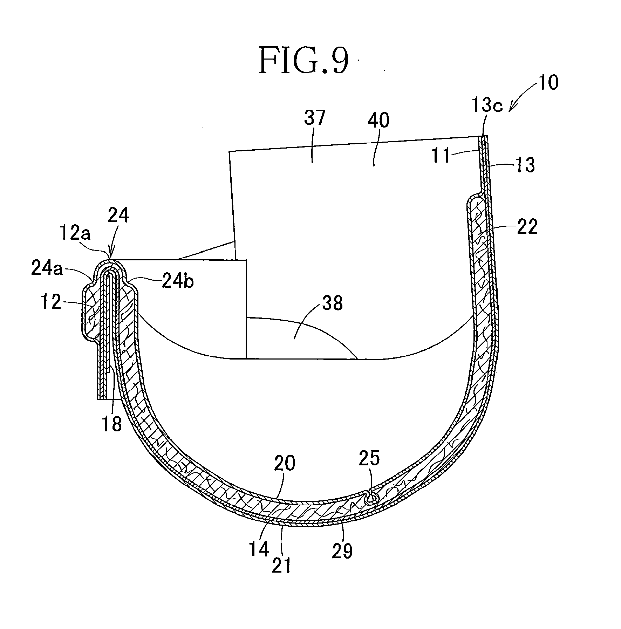

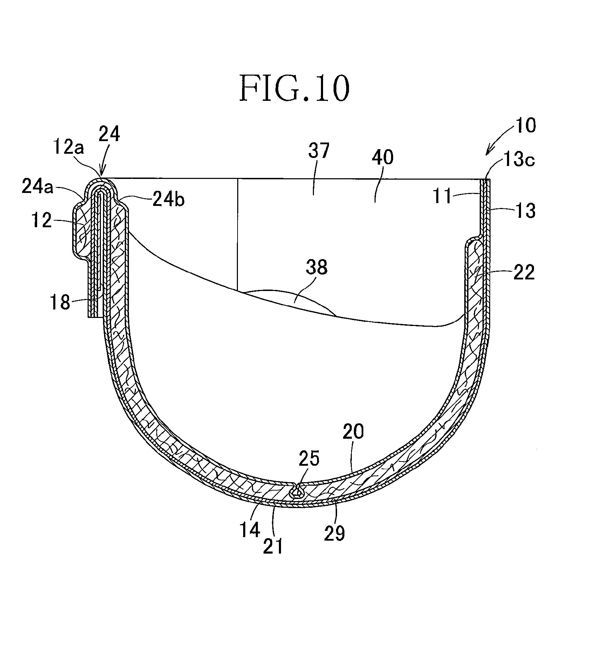

{Fig. 1} Fig. 1 is a perspective view illustrating a disposable diaper as an example of a disposable wearing article according to a first embodiment of the present invention.
{Fig. 2} Fig. 2 is a partially cutaway developed plan view illustrating the diaper as has been developed in a front-back direction and viewed from an inner surface thereof.
{Fig. 3} Fig. 3 is a sectional view taken along line III-III in Fig. 1.
{Fig. 4} Fig. 4 is a partially scale-enlarged diagram illustrating a zone designated by IV in Fig. 2.
{Fig. 5} Fig. 5 is a sectional view taken along line V-V in Fig. 4.
{Fig. 6} Fig. 6 is a perspective view illustrating the diaper according to another embodiment.
{Fig. 7} Fig. 7 is a longitudinal sectional view taken along line VI-VI in Fig. 6.
{Fig. 8} Fig. 8 is a sectional view similar to Fig. 3, illustrating the diaper according to a second embodiment of the present invention.
{Fig. 9} Fig. 9 is a sectional view similar to Fig. 7, illustrating the diaper according to a third embodiment of the present invention.
{Fig. 10} Fig. 10 is a sectional view similar to Fig. 7, illustrating the diaper according to the third embodiment of the present invention in a worn state.

### {Description of Embodiments}

Fig. 1 is a perspective view illustrating a disposable diaper 10 as an example of a disposable wearing article according to a first embodiment of the present invention, Fig. 2 is a partially cutaway developed plan view illustrating the diaper 10 as has been developed in a front-back direction and viewed from an inner surface thereof, Fig. 3 is a sectional view taken along line III-III in Fig. 1, Fig. 4 is a partially scale-enlarged diagram illustrating a zone designated by IV in Fig. 2, Fig. 5 is a sectional view taken along line V-V in Fig. 4, Fig. 6 is a perspective view illustrating the diaper according to another embodiment and Fig. 7 is a longitudinal sectional view taken along line VII-VII in Fig. 6.

As illustrated in Figs. 1 through 4, the diaper 10 has a longitudinal axis P and a transverse axis Q and includes a diaper chassis 11 which includes, in turn, a skin-contactable surface and a non-skin-contactable surface opposite to the skin-contactable surface, a front waist region 12, a rear waist region 13 and a crotch region 14 extending between the front and rear waist regions 12, 13, and a pair of tape fastener tabs 15, 16 extending outward in a direction of the transverse axis Q from lateral edges 13a, 13b of the rear waist region 13 of the diaper chassis 11, respectively. The tape fastener tabs 15, 16 are provided on the respective skin-contactable surfaces thereof with first fastening elements 17 each defined by a group of hooks constituting a mechanical fastener. A second fastening element 18 extending in the direction of the transverse axis Q and defined by a group of loops constituting a mechanical fastener is attached to an outer surface of the front waist region 12 with a hot melt adhesive (not shown) so that the respective first fastening elements 17 of the tape fastener tabs 15, 16 may be detachably fastened to this second fastening element 18.

The diaper chassis 11 includes a liquid-permeable topsheet 20 formed of, for example, a fibrous nonwoven fabric, a porous plastic film or a laminate thereof and lying on the side of the skin-contactable surface, a liquid-impermeable backsheet 21 formed of, for example, a breathable plastic film, a fibrous nonwoven fabric or a laminate thereof and lying on the side of the non-skin-contactable surface so as to define an outer surface of the diaper 10 and an absorbent core 22 formed of a mixture of fluff pulp and superabsorbent polymer particles and interposed between the topsheet 20 and the backsheet 21. The topsheet 20 and the backsheet 21 extend outward beyond a periphery of the absorbent core 22 and are bonded to each other with a hot melt adhesive (not shown) applied to an inner surface of at least one of the top- and backsheets 20, 21 along the respective extensions. Though not illustrated, it is possible to wrap the absorbent core 22 with a diffusion sheet such as tissue paper.

The topsheet 20 may be formed of, for example, an air-through fibrous nonwoven fabric, a spunbonded fibrous nonwoven fabric or a point bonded nonwoven fabric having a mass per unit area in a range of 20 to 50 g/m², preferably in a range of 30 to 40 g/m² and a fiber density in a range of 0.01 to 0.04 g/m², preferably in a range of 0.025 to 0.035 g/cm³. The backsheet 21 may be formed of, for example, a spunbonded nonwoven fabric having a mass per unit area in a range of 15 to 40 g/m², preferably in a range of 25 to 35 g/m² and a fiber density in a range of 0.06 to 0.1 g/cm³, preferably in a range of 0.07 to 0.09 g/cm³.

The absorbent core 22 is formed in the front waist region 12 with a fold guiding zone 24 extending in the direction of the transverse axis Q and in the crotch region 14 with a crotch fold guiding zone 25 extending on and along the transverse axis Q. The fold guiding zone 24 and the crotch fold guiding zone 25 are provided in the form of grooves created as a result of dimensioning these zones in the absorbent core 22 in a direction Z (i.e., in a three-dimensional direction) so as to have a thickness smaller than that of the remaining zone as measured in the diaper chassis 11 in its flatly developed state (See Fig. 3). When the diaper 10 is put on the wearer's body, the absorbent core 22 formed with the fold guiding zone 24 and the crotch fold guiding zone 25 facilitate the diaper 10 to be doubled up in its front-back direction by the intermediary of the crotch fold guiding zone 25 and, as will be described later in more details, makes it possible to fold the front waist region 12 outward by the intermediary of the fold guiding zone 24. Even if, in addition to the crotch fold guiding zone 25, the crotch region is formed with one or more creases which would otherwise potentially induce doubling up of the diaper and such zones would be spaced apart from the wearer's body to cause leakage of body waste, the crotch fold guiding zone 25 primarily induces doubling up of the diaper 10 and thereby such an anxious situation can be avoided. As illustrated in Figs. 2 and 3, opposite ends 24a, 24b of the fold guiding zone 24 in the absorbent core 22 are appropriately curved and the wearer's skin should not be irritated even when this fold guiding zone 24 comes in contact with the wearer's skin. In this regard, while the fold guiding zone 24 is illustrated to be formed at a level adjacent to the lower end of the front waist region 12, in other words, formed so that the front waist region 12 as a whole may be folded outwardly, it is also possible to form the fold guiding zone 24 adjacent to a middle of the front waist region 12 as viewed in the direction of the longitudinal axis P so that only part of the front waist region 12 may be folded outwardly.

The fold guiding zone 24 is provided in the form of a groove having a given width, i.e., a dimension I in the direction of the longitudinal axis P, and the dimension I is preferably 10 mm or more in order to ensure advantageous effects as described later of the present invention. The fold guiding zone 24 is located to overlap a lower end 18a of the second fastening element 18 provided on the outer surface of the diaper chassis 11 and a zone on the outer surface of the diaper chassis 11 corresponding to the fold guiding zone 24, more specifically, corresponding to the lower end 18a of the second fastening element 18 and adjacent thereto is formed with a fold guiding line 28 making it possible the wearer and/or the care person to recognize visually the location of the fold guiding zone 24 from the outside of the diaper 10 (See Fig. 1). It is also possible to provide the fold guiding line 28 in the form of coloring which is distinguished from that of the second fastening element 18 or a tape strip affixed to the location corresponding to the fold guiding zone 24 in order that the wearer and/or the care person can visually recognize the location of the fold guiding zone 24 without any difficulty.

While both the fold guiding zone 24 and the crotch fold guiding zone 25 are provided in the form of the grooves defined by concaving the corresponding zones on the skin-contactable surface of the absorbent core 22 in the embodiment illustrated in Fig. 3, it is also possible to form such grooves by concaving the corresponding zones of the non-skin-contactable surface of the absorbent core 22. Further it is also possible to form the fold guiding zone 24 as well as the crotch fold guiding zone 25 by cutting away corresponding portions of the absorbent core 22 or compressively deforming the absorbent core 22 in the corresponding zones thereof, respectively. In addition, it is also possible to form both the fold guiding zone 24 and the crotch fold guiding zone 25 by differentiating a density of the absorbent core 22 so as to be lower in these zones than in the remaining zone while keeping a thickness of the absorbent core 22 uniform over its entire range.

A leakage-barrier sheet 29 formed of a liquid-impermeable but breathable plastic film is interposed between the topsheet 20 and the backsheet 21 and secured with a hot melt adhesive (not shown) applied to either one of these two sheets 20, 21. On the skin-contactable surface A of the diaper 10, there is provided, outboard of the absorbent core 22 as viewed in the direction of the transverse axis Q, a pair of barrier sheets 30, 31 symmetrically about the longitudinal axis P.

The diaper chassis 11 further includes front and rear end portions 32, 33 opposite to each other in the direction of the longitudinal axis P and extending in the direction of the transverse axis Q and lateral portions 34, 35 opposite to each other in the direction of the transverse axis Q and extending in the direction of the longitudinal axis P across the crotch region 14. In the course of putting the diaper 10 on the wearer's body, the respective first fastening elements 17 carried by the pair of tape fastener tabs 15, 16 may be engaged with the second fastening element 18 provided on the non-skin-contactable surface of the front waist region 12 to join the respective lateral edges of the front and rear waist regions 12, 13 to each other and thereby to define a waist-opening 37 and a pair of leg-openings 38 (See Fig. 1).

The front and rear end portions 32, 33 are respectively formed of portions of the topsheet 20, the leakage-barrier sheet 29, the backsheet 21 and the barrier-sheets 30, 31 extending outward in the direction of the longitudinal axis P beyond the front and rear ends of the absorbent core 22 overlapped with one another. The lateral portions 34, 35 are respectively formed of portions of the topsheet 20, the leakage-barrier sheet 29, the backsheet 21 and the barrier-sheets 30, 31 extending outward in the direction of the transverse axis Q beyond the lateral edges of the absorbent core 22 overlapped with one another. In the front and rear waist regions 12, 13, the backsheet 21 and the barrier sheets 30, 31 extend further outward in the direction of the transverse axis Q beyond respective lateral edges of the topsheet 20 and the leakage-barrier sheet 29 defining the lateral portions 34, 35 and then overlapped with each other to define front and rear side flaps 39, 40, respectively. Secured portions 15a, 16a of the paired tape fastener tabs 15, 16 are interposed between respective laterals of the backsheet 21 and the barrier sheet 30 cooperating with each other to define the rear side flaps 40 and secured between the respective laterals with a hot melt adhesive (not shown) applied to respective inner surfaces of these two sheets. Respective free portions 15b, 16b of the tape fastener tabs 15, 16 extending in the direction of the transverse axis Q from side edges of the respective rear side flaps 40 (corresponding to the lateral edges 13a, 13b of the rear waist region 13) are provided on the skin-contactable surface thereof with the first fastening elements 17.

In the first fastening system composed of the first fastening elements 17 and the second fastening element 18, the first fastening elements 17 may be defined by adhesive-coated zones instead of the respective groups of hooks so long as the respective first fastening elements 17 have a desired peel strength. In this case, it is also possible to cover the respective surfaces of the first fastening elements 17 with separators coated with silicone to protect the first fastening elements 17.

The tape fastener tabs 15, 16 are preferably formed of sheet material which has a relatively high stiffness and tensile strength such as a plastic film, a fibrous nonwoven fabric, a laminate thereof or craft paper. Alternatively, the first fastening elements 17 provided on the rear side flaps 40 may be detachably engaged with the second fastening element 18 without use of the tape fastener tabs 15, 16 so long as the desired effects of the present invention can be ensured.

As illustrated in Fig. 4, the skin-contactable surface of the topsheet 20 in the front end portion 32 is formed with a third fastening element 45 with which the first fastening elements 17 can be engaged and this third fastening element 45 is embossed in such a manner that a plurality of first and second concave lines 43, 44 extending obliquely with respect to the longitudinal axis P and the transverse axis Q intersect with each other in a lattice-pattern. The third fastening element 45 is formed in a zone surrounded by the respective lateral portions 34, 35 of the fold guiding zone 24 and the front waist region 12. While shape, size and intersecting angle of the first concave line 43 and the second concave line 44 may be appropriately selected, the intersecting angle of the first concave lines 43 and the second concave lines 44 cooperating together to define the third fastening element 45 is about 90° and each of lattices 47 defined by intersection between the first concave lines 43 and the second concave lines 44 has a size (area) in a range of about 9.0 to about 12.0 mm². The plural lattices 47 defined by the first and second concave lines 43, 44 intersecting with each other in this manner ensure that the fibers constituting the topsheet 20 should not be fluffed up when the first fastening elements 17 are engaged with the surface of the topsheet 20 and, in addition, make it possible to adjust an engagement strength of the second fastening system to an appropriate level, This is because it is rather difficult for the first fastening elements 17 to be firmly engaged with the embossed zone. Further, the third fastening element 45 formed with use of embossing facilitates for the wearer and/or the care person to recognize the location of the third fastening element 45 visually.

As illustrated in Fig. 5, the topsheet 20 is formed on the skin-contactable surface with a plurality of ridges 50 and a plurality of grooves 51 each defined between each pair of the adjacent ridges 50. The fibers constituting the topsheet 20 are oriented at random in all directions (including three-dimensional direction) and such a convex-concave pattern may be obtained by gas jets, water jets or steam jets from nozzles arranged above a continuous fibrous web adapted to form the topsheet 20 or subjecting the continuous fibrous web to press working or gear working. The convex-concave pattern of the topsheet 20 further facilitates the third fastening element defined by the first and second concave lines 43, 44 to be engaged with the first fastening elements 17 and, in the topsheet 20 as a whole, the ridges 50 primarily come in contact with the wearer's body while the grooves 51 define air passages extending in the direction of the longitudinal axis P through which moisture generated within the diaper may be exhausted outside the diaper. In this way, a high air-permeability is ensured.

It is possible to reorient the fibers constituting the fibrous web so that a fiber density may be adjusted so as to be lower in the grooves 51 than in the ridges 50. In this case, body waste quickly moves through the grooves 51 to the absorbent core 22 and, as a result, it is possible to prevent a discomfort feeling which otherwise would be experienced by the wearer due to wet condition of the topsheet 20. It is also possible to regulate an amount of gas jets and thereby to form the respective grooves 51 with through-holes intermittently arranged so that loose passage of a relatively high viscosity may quickly move downward through these through-holes.

As has previously been described, the topsheet 20 has the convex-concave pattern on the skin-contactable surface thereof and, for this reason, the topsheet 20 can be engaged with the first fastening elements 17 to fulfill the function as the third fastening element 45 even when the topsheet 20 is not embossed to form the first and second concave lines 43, 44. It is possible to provide a fibrous nonwoven fabric prepared separately from the topsheet 20 and having a convex-concave pattern similar to that of the topsheet 20 in the present embodiment as the third fastening element 45 or to use such a fibrous nonwoven fabric prepared separately from the topsheet 20 without subjecting this fibrous nonwoven fabric to embossing. Further, it is also possible to use the topsheet 20 formed of, for example, a spunbonded fibrous nonwoven fabric of long fibers neither having the convex-concave pattern nor subjected to embossing as the third fastening element 45.

Referring again to Fig. 2, the barrier sheets 30, 31 respectively have proximal edges 54 partially constituting the lateral portions 34, 35, front and rear secured end portions 55, 56 secured to the skin-contactable surface of the topsheet 20 and the backsheet 21 in the front and rear waist regions 12, 13, respectively, and distal edges 57 defined by inner side edge portions of the barrier sheets 30, 31 folded inwardly so as to extend in the direction of the longitudinal axis P between the front and rear secured end portions 55, 56. Outboard of the lateral edges of the leakage-barrier sheet 29 as viewed in the direction of the transverse axis X, three strand or string leg elastic elements 58 extending in the direction of the longitudinal axis P are contractibly attached between the proximal edges 54 of the respective barrier sheets 30, 31 and the lateral portions of the backsheet 21 with a hot melt adhesive (not shown), respectively. The distal edges 57 are respectively provided with two strand or string cuff elastic elements 59 contractibly attached thereto. During use of the diaper 10, the distal edges 57 are spaced away from the skin-contactable surface of the topsheet 20 under contraction of the cuff elastic elements 59 so as to form a pair of leakage-barrier cuffs adapted to prevent body waste from leaking sideways.

Fig. 6 is a diagram illustrating a variant of the diaper 10 according to the first embodiment and Fig. 7 is a sectional view taken along line VII-VI in Fig. 6.

As illustrated in Fig. 6, the front waist region 12 may be folded outwardly along the fold guiding zone 24 and then the first fastening elements 17 formed on the skin-contactable surface of the front end portion 32 may be engaged with the third fastening element 45 to put the diaper 10 having the front waist region 12 folded onto the non-skin-contactable surface of the crotch region 14 on the wearer's body. In this state, the wearer's abdominal region is kept to be exposed and, particularly when the wearer is a newborn baby inclusive of an immature baby, abdominal respiration of such a baby should not be impeded due to a tightening effect of the front waist region 12. Particularly for newborn babies, a navel and the vicinity thereof is still immature and such region might be irritated due to the tightening effect of the front waist region 12 even when such tightening effect is relatively slight. However, such undesirable situation can be avoided by the present invention.

Below the navel, the first fastening elements 17 are engaged with the third fastening element 45 after the front waist region 12 has been folded and, for this reason, the diaper 10 can be stabilized on the wearer's body in a lower abdominal region thereof even if the front waist region 12 is not in contact with the wearer's abdominal region. In addition, the front waist region 12 should not be unfolded during use of the diaper 10.

Besides, during use of the diaper 10 utilizing the second fastening system as illustrated in Figs. 6 and 7, the front waist region 12 is folded onto the non-skin-contactable surface of the crotch region 14 and, in consequence, the rear waist region 13 and the rear side flaps 40 thereof are pulled obliquely forward to a small extent so that the tape fastener tabs 15, 16 may be obliquely attached to the second fastening element 18. In this state, the leg-openings 38 become reduced in size compared to the state (See Fig. 3) in which the diaper 10 is put on the wearer's body utilizing the first fastening system so that the vicinities of the respective leg-openings 38 may be closely fitted about the legs of a newborn baby having a relatively small build and thereby it is possible to prevent body waste from leaking beyond the peripheries of the leg-openings 38.

According to the present invention, as has been described above, either one of the first and second fastening systems may be selectively utilized depending on the body size of the wearer. Specifically, the first fastening system may be utilized for infants of an average body size and the second fastening system may be utilized for newborn babies inclusive of immature babies whose body size is smaller than that of the average infant to put the diaper 10 in close contact with the wearer's body. In this way, the size adjustment of the diaper 10 is relatively easy.

As has previously been described also, the dimension I in the direction of the longitudinal axis P of the fold guiding zone 24 is 10 mm or more, in other word, the fold guiding zone 24 has a given width dimension. For this reason, the fold line formed along the fold guiding zone 24 and the vicinity thereof is not sharp but gently curved and an upper end 12a of the front waist region 12 defined by the fold guiding zone 24 should not dig into the wearer's abdomen even when the absorbent core 22 has a required stiffness.

Besides, the front waist region 12 is folded outwardly and, in consequence, the part of the absorbent core 22 lying in the front waist region 12 and the part of the absorbent core 22 lying in the crotch region 14 overlap with each other in the front-back direction of the diaper 10. As a result, cushioning properties of the crotch region 14 are improved and, in addition, it is facilitated to pinch the front waist region 12 together with the crotch region 14 with one's fingers to pull up the diaper 10 when the diaper 10 is displaced when the diaper 10 is put on the wearer's body or during use of the diaper 10.

### <Second Embodiment>

Fig. 8 is a sectional view similar to Fig. 7, illustrating a second embodiment of the present invention. A basic arrangement of a diaper 10 according to the second embodiment is the same as that of the diaper 10 according to the first embodiment and therefore only an aspect of the second embodiment distinguished from the first embodiment will be described below.

As illustrated in Fig. 8, the absorbent core 22 is not present in the fold guiding zone 24 according to this embodiment. The absorbent core 22 is composed of a separated part 22A lying in the front waist region 12 and a main part 22B extending across the crotch region 14 into the rear waist region 13. The absorbent core 22 separated off into the two parts further facilitates the front waist region 12 to be folded along the fold guiding zone 24 and, at the same time, makes it possible to prevent the amount of bodily fluids once absorbed by the main part 22B of the absorbent core 22 lying in the crotch region 14 from being diffused to the separated part 22A lying outboard of the crotch region 14 and thereby the wearer's garment is unlikely to be soiled with bodily fluids.

### <Third Embodiments>

Fig. 9 is a sectional view similar to Fig. 7, illustrating a diaper 10 according to a third embodiment of the present invention and, Fig. 10 is a sectional view similar to Fig. 7, illustrating the diaper 10 according to the third embodiment of the present invention in the worn state. A basic arrangement of the diaper 10 according to the third embodiment is the same as that of the diaper 10 according to the first embodiment and therefore only an aspect of the third embodiment distinguished from the first embodiment will be described below.

According to this embodiment, as illustrated in Fig. 9, the crotch fold guiding zone 25 extends in the direction of the transverse axis Q across the absorbent core 22 in the crotch region 14 at a location biased to the rear waist region 13. With such an arrangement, the front waist region 12 may be folded by the intermediary of the fold guiding zone 24, then the rear waist region 14 may be pulled down to a level at which the crotch fold guiding zone 25 is opposed to the middle of the wearer's crotch and then the diaper 10 may be folded in the front-back direction along the crotch fold guiding zone 25 to ensure that a height of the front waist region 12 folded by the intermediary of the fold guiding zone 24 and a height of the rear waist region 13 are substantially the same, i.e., a position of the upper end 12a of the front waist region 12 and a position of an end 13c of the rear waist region are substantially in alignment with each other (See Fig. 10). In such situation, the front and rear waist regions 12, 13 are substantially on a same level and the wearer's abdominal region is kept to be exposed. In consequence, the abdominal region might be compressed and, in addition, the diaper 10 can be put on the wearer having a relatively small body size (for example, newborn baby or immature baby) with the diaper fitted fully about the waist and/or the lower abdominal region. Specifically, the leg-openings 38 of the diaper 10 are defined to be smaller when putting the diaper on the wearer's body utilizing the second fastening system than when putting the diaper on the wearer's body utilizing the first fastening system as has previously described. Therefore, the diaper 10 may be put on the wearer's body utilizing the second fastening system as needed basis to fit the peripheries of the leg-openings 38 of the diaper 10 about the legs of a newborn baby or an immature baby which are thinner than those of average infants.

According to this embodiment, in addition to the advantageous effect such that the diaper 10 may be folded so as to be substantially the same and thereby fit of the leg-openings' peripheries about the wearer's legs may be further improved, it is also possible to, after the second fastening system has been released for the purpose of correction, put the diaper 10 again on the wearer's body to ensure a desired fit of the peripheries of the leg-openings 38 about the wearer's legs. Thereby the diaper can be properly fitted to the wearer's body even if the wearer's body size is relatively small. In this manner, the diaper 10 can be put on the wearer's body in three wearing modes, i.e., a wearing mode utilizing the first fastening system (first wearing mode), a wearing mode utilizing the second fastening system so that the heights of the front and rear waist regions 12, 13 are different from each other (second wearing mode) and a wearing mode utilizing the second fastening system so that the heights of the front and rear waist regions 12, 13 are substantially the same (third wearing mode). These three wearing modes may be selectively adopted depending on the wearer's body size and thereby a size control may be easily achieved.

Usually, a plurality of the diapers 10 are packaged in such a manner that the individual diapers 10 are respectively doubled up and then stacked on one another. During storage of the diapers 10 in such a packaged state, the individual diapers 10 are often formed with creases along the doubled up zones, particularly in the absorbent cores 20 having a stiffness higher than that in the sheet members. If such creases are formed in the zone of the front waist region 12 in each of the diapers 10 opposed to the urinary organ of the wearer, the fit in this zone might be deteriorated and eventually cause leakage of body waste. However, the crotch fold guiding zone 25 is formed in a zone of the absorbent core 22 in the crotch region 14 biased to the rear waist region 13 as illustrated in Fig. 9 and therefore the creases are formed in the vicinity of such location. Consequently, in vicinities of the creases formed due to doubling up the diaper at a distance forward from the zone biased to the rear waist region 13, the diaper 10 is fitted to the vicinity of the wearer's urinary organ and body waste should not leak out.

### {Reference Signs List}

| | |
|---|---|
| 10 | disposable wearing article |
| 11 | diaper chassis |
| 12 | front waist region |
| 13 | rear waist region |
| 13a, 13b | lateral edges of rear waist region |
| 14 | crotch region |
| 15, 16 | tape fastener tabs |
| 17 | first fastening elements |
| 18 | second fastening element |
| 22 | absorbent core |
| 24 | fold guiding zone |
| 25 | crotch fold guiding zone |
| 28 | fold guiding line |
| 45 | third fastening element |
| P | longitudinal axis |
| Q | transverse axis |

## Claims

1. A disposable wearing article having a longitudinal axis and a transverse axis, the article comprising:
a chassis including a skin-contactable surface, a non-skin-contactable surface opposite to the skin-contactable surface, a front waist region, a rear waist region and a crotch region extending in a direction of the longitudinal axis between the front and rear waist regions; and
a fastening system adapted to join respective lateral edges of the front and rear waist regions, wherein:
the chassis is provided with an absorbent core extending across the crotch region into the front and rear waist regions;
the absorbent core is formed with a fold guiding zone extending in the direction of the transverse axis in the front waist region; and
the fastening system includes a first fastening system and a second fastening system, wherein
the first fastening system includes first fastening elements provided on the respective skin-contactable surfaces of a pair of tape fastener tabs extending outward in the direction of the transverse axis from lateral edges of the rear waist region and a second fastening element formed on the non-skin-contactable surface of the front waist region, and
the second fastening system includes the pair of the first fastening elements and a third fastening element formed on the skin-contactable surface of the front waist region.

2. The wearing article according to claim 1, wherein the absorbent core in the. crotch region is formed with a crotch fold guiding zone extending along the transverse axis.

3. The wearing article according to claim 2, wherein the crotch fold guiding zone is formed on the absorbent core in the crotch region at a location biased toward the rear waist region.

4. The wearing article according to claim 2, wherein the fold guiding zone and the crotch fold guiding zone are provided in the form of grooves.

5. The wearing article according to claim 1, wherein the absorbent core is not present in the fold guiding zone.

6. The wearing article according to claim 1, wherein the third fastening element is formed by embossing a fibrous nonwoven fabric sheet defining the skin-contactable surface of the front waist region.

7. A disposable wearing article having a longitudinal axis and a transverse axis, the article comprising:
a chassis including a skin-contactable surface, a non-skin-contactable surface opposite to the skin-contactable surface, a front waist region, a rear waist region and a crotch region extending in a direction of the longitudinal axis between the front and rear waist regions; and
a fastening system adapted to join respective lateral edges of the front and rear waist regions, wherein:
the chassis is provided with an absorbent core extending across the crotch region into the front and rear waist regions;
the absorbent core is formed with a fold guiding zone extending in the direction of the transverse axis in the front waist region;
the fastening system includes a first fastening system and a second fastening system, wherein
the first fastening system includes first fastening elements provided on the respective skin-contactable surfaces of a pair of tape fastener tabs extending outward in the direction of the transverse axis from lateral edges of the rear waist region and a second fastening element formed on the non-skin-contactable surface of the front waist region, and
the second fastening system includes the pair of the first fastening elements and a third fastening element formed on the skin-contactable surface of the front waist region; and
the second fastening element is located on the non-skin-contactable surface of the front waist region so as to be closer to the waist-opening than to the fold guiding zone and a sub-region in the front waist region extending outward from the fold guiding zone in the direction of the transverse axis may be folded outwardly to engage the first fastening elements with the third fastening element.

8. The wearing article according to claim 7, wherein the absorbent core in the crotch region is formed with a crotch fold guiding zone extending along the transverse axis.

9. The wearing article according to claim 8, wherein the crotch fold guiding zone is formed on the absorbent core in the crotch region at a location biased toward the rear waist region.

10. The wearing article according to claim 8, wherein the fold guiding zone and the crotch fold guiding zone are provided in the form of grooves.

11. The wearing article according to claim 7, wherein the absorbent core is not present in the fold guiding zone.

12. The wearing article according to claim 7, wherein the third fastening element is formed by embossing a fibrous nonwoven fabric sheet defining the skin-contactable surface of the front waist region.

13. The wearing article according to claim 7, wherein the non-skin-contactable surface of the front waist region is provided in a zone corresponding to the fold guiding zone with a fold guiding line adapted to be visually recognized from the outside.
